# EUROPEAN PATENT SPECIFICATION

(11) **EP 0 812 213 B1**
(45) Date of publication and mention of the grant of the patent: **13.08.2003**
(21) Application number: 96902194.8
(22) Date of filing: 21.02.1996
(51) Int. Cl.: A61K 47/36, A61K 9/00

(54) **GEL FORM OF A VACCINE**
IMPFSTOFF IN GELFORM
VACCIN SE PRESENTANT SOUS FORME DE GEL

(30) Priority: 21.02.1995 US 390956
(43) Date of publication of application: 17.12.1997
(73) Proprietor: Lee, Eng-Hong, Rockwood, Ontario N0B 2K0 (CA)
(72) Inventor: Lee, Eng-Hong, Rockwood, Ontario N0B 2K0 (CA)
(74) Representative: Brooks, Nigel Samuel
(86) International application number: CA9600100
(87) International publication number: WO96025951

(56) References cited:
- EP-A- 0 243 548
- WO-A-85/00752

## Description

The present invention relates to a gel form for administration of live vaccines and supplements to poultry hatchlings.

### BACKGROUND OF THE INVENTION

At the present time, poultry hatchlings, within the first few days of life, are required to be immunized against various diseases and the type of vaccine used for each disease dictates its method of administration. Attenuated vaccines are usually administered in the hatchery by injection at the time of sorting of the hatchlings from the hatching incubator into holding or transporting trays. Live vaccines are more commonly administered once the hatchlings are established in their brooding trays in the form of aqueous suspensions either sprayed on feed or added to the drinking water.

One example of a live vaccine is that used to immunize poultry against coccidiosis caused by protozoa of the genus Eimeria. Coccidiosis is a very common disease of poultry and there are several species of Eimeria which are known to cause such disease. The symptoms and severity of the disease are dependent upon the species of Eimeria with which the bird is infected with E. tenella, E.acervulina and E.maxima being three of the most prevalent species. At the present time, the protection of poultry against coccidiosis involves two possible methods - use of anticoccidials as feed additives or immunization using a coccidiosis vaccine with immunization being increasingly the preferred route. Coccidiosis vaccines are at present comprised of virulent strains of coccidia in a suitable carrier for administration, the coccidia being capable of causing a mild form of the disease and selected to be very anticoccidial susceptible.

One common method of immunization against coccidiosis involves the use of on-feed spray administration while the birds are feeding from flats or other containers. A vaccine comprising oocysts of Eimeria species in a water based carrier is sprayed onto the feed to be provided to the hatchlings. The use of on-feed spray administration requires large doses of oocysts and uniform exposure of the flocks to the vaccine cannot always be achieved.

Vaccine may also be administered through the use of water proportioning systems including automatic fountains and automatic water medicator or proportioners. However, given the particulate nature of coccidiosis vaccines, it is doubtful that the vaccine may actually make it to the end of the water line, resulting in uneven exposure of the flock. Additionally, administration of the vaccine through the water proportioning system requires that after administration of the vaccine, the proportioning system be thoroughly cleaned to remove any residual vaccine.

Poultry hatchlings are at present generally immunized against coccidiosis after they are 3 to 4 days old. By delaying this immunization until this point in time, the chicks are fully in the feeding cycle and are able to become immunized through the use of the vaccine either on the feed or through the drinking water.

It would, however, be advantageous to immunize the hatchling at an earlier stage, preferably within the hatchery. By doing this, lower doses of oocysts may be required for immunization and the oocysts may be administered as part of the drinking water which is used as the inducement for the commencement of the feeding cycle. The administration of vaccine in the drinking water requires that the oocysts remain suspended to provide for even exposure of the flock. One solution to this has been proposed by the present applicant in Canadian Patent 1,204,057, which involves suspending the oocysts in a 1.5% carrageenan solution. While this method has numerous advantages, such as reduced levels of oocysts necessary to provide immunization as well as ease of administration, there is still a drawback in that the provision of open watering systems to hatchlings could result in the liquid being spilled or wetting the hatchlings which could potentially affect the health of the hatchlings, especially in cold weather and during transportation.

There thus remains a need for a simplified vaccine for administration to day-old hatchlings in the hatchery, which provides adequate protection of the flock while reducing potential problem areas.

### SUMMARY OF THE INVENTION

The present invention provides for a gel form of a live vaccine for administration of live vaccine to poultry hatchlings. The gel form comprises approximately 1 to 2.5 percent of an edible polysaccharide temperature setting gelling agent having suspended therein sufficient levels of a live vaccine to provide for immunization of a poultry hatchling flock.

In an aspect of the invention, there is provided a method of preparing a gel for immunizing a poultry flock utilizing a live vaccine of viable organisms, the method comprising preparing a gel form of a live vaccine by preparing a suspension of the viable organisms in cold water, dissolving an edible polysaccharide temperature setting gelling agent in an aqueous solution at or above the melting point of the polysaccharide gelling agent, mixing the organism suspension and the gelling agent solution, pouring the mixture into a suitable container then allowing the mixture to cool and gel to produce a gelled live vaccine comprising from about 1 to about 2.5 percent of an edible polysaccharide gel having suspended therein sufficient levels of the organism to provide immunization to the flock and allowing the poultry flock to feed from the gelled vaccine suspension.

### DETAILED DESCRIPTION OF THE PREFERRED EMBODIMENTS

In a poultry hatchery, at the time of emergence of the hatchlings from their shells in the incubation trays, they are generally examined for defects, immunized with poultry vaccines by injection and then sorted by sex and placed into holding or transporting trays. At this time they may also be administered aqueous based live vaccines through a suitable means such as gavage (direct oral administration). Once in the brooding trays, the chicks may also be administered live vaccine by means of the watering system or by means of on feed spray. Aqueous-based live vaccines are generally particulate in nature and if administered in the watering system, the vaccines should be provided in a composition which will maintain a relatively uniform suspension of the organisms in the vaccine. This is particularly true for coccidiosis vaccines which consist of relatively large oocysts of Eimeria species. Coccidiosis vaccines are usually administered orally for immunizing domestic animals of the avian species from coccidiosis, the vaccine having oocysts of at least one coccidium in relation to which the immunization is desired.

The present invention in a preferred embodiment provides for a gel form of a coccidiosis vaccine in which the oocysts of the coccidium are diluted and suspended in a gel form which results in maintaining an uniform suspension of the oocysts and consequently, relatively uniform infection of the flock by the oocysts. The gel form of the present invention is particularly useful with Eimeria species, more particularly with Eimeria tenella or other species such as Eimeria necatrix, Eimeria hagani, Eimeria acervulina, Eimeria maxima, Eimeria brunetti, Eimeria proecox, Eimeria mivati and Eimeria mitis are also useful. The method of obtaining each of these species is well-known by those skilled in the art. Two or more species of Eimeria can be used simultaneously and in the practice of the present invention, it is generally not necessary to use more than six species together in the same suspension.

The gel form vaccine of the present invention provides for an easy to handle method of vaccinating poultry hatchlings in the hatchery and is, therefore, suitable for general hatchery workers without any special expertise required. The gel form is produced utilizing an edible temperature setting polysaccharide gel, preferably a low temperature setting alginate or carrageenan gel, most preferably the kappa carrageenan gel sold as refined carrageenan Bengel MB 910®, a water soluble kappa-type carrageenan extracted from the red algae Eucheuma cottonii.

The gel form of the vaccine is prepared by dissolving the gel powder in water at a suitable temperature to effect complete dissolution of the polysaccharide powder. The powder is added to the water at a concentration such that, when mixed with the oocyst suspension and allowed to gel, a relatively soft gel results. Typically, the dissolved gel powder and oocyst suspension are mixed in a ratio of about 1:1 (v:v) to prepare the gel form of the vaccine. Suitable such gel forms have been found to have a final concentration of the edible polysaccharide in the gel form of between about 1 and 2.5 percent, preferably between about 1 and 2 percent, more preferably between about 1.25 and 1.75 percent and most preferably about 1.25 percent. Thus preferably, a dissolved polysaccharide gel solution of about 2 to 5 percent, preferably about 2 to 4 percent, more preferably about 2.5 to 3.5 percent, most preferably about 2.5 percent, is mixed with an equal volume of a suspension of oocysts and the mixture allowed to gel at a suitable temperature.

The gel form vaccine of the present invention has sufficient levels of the oocysts to provide immunization to the flock. It has been found that each hatchling will consume about 0.5 to 1.5 ml of the gel and the concentration of the oocysts in the gel should be such as to provide sufficient organisms in this typical volume to immunize the hatchling. It has been found that between about 50 and 1,000 oocysts per bird provides adequate protection and so it is preferred if the gel form of the vaccine has between about 100 and 500 oocysts per ml of gel, to provide for proper immunization of the flock. Preferably, the gel form of the vaccine contains between about 200 and 300 oocysts per ml of gel, most preferably about 250 oocysts per ml of gel. As the gel form is prepared by mixing the dissolved polysaccharide powder with the oocyst suspension in equal volumes, preferably one volume of a 2 to 5 % polysaccharide gel solution is mixed with an equal volume of oocyst suspension containing between about 200 and 1,000 oocysts per ml, more preferably one volume of a 2 to 4 percent polysaccharide solution with an equal volume of oocyst suspension containing between about 400 and 600 oocysts per ml, most preferably a 2.5 percent solution of polysaccharide is mixed with an equal volume of oocyst suspension containing about 500 oocysts per ml.

The use of this edible temperature setting polysaccharide form does not require the use of catalyst systems. Catalyst systems such as CMC generally require more careful handling particularly during the gelling process. If the concentrations of catalyst and gel compound are not properly adjusted, the gelling of the solutions may occur too quickly and may not permit sufficient mixing to provide for a uniform suspension of the parasites. The use of the edible polysaccharide gels of the present invention results in a gel which preferably forms in about 3 to 5 minutes at 4°C, maintains the vaccine organisms in uniform suspension and allows for more uniform exposure of the poultry hatchlings to the vaccine organisms. The low content of the edible gum in the gel form means that approximately 95 percent or more of the gel form is water which can aid in the hydration of the bird and induce the feeding response. The gel form has other advantages over liquid suspensions in that the gel form will not wet the bird and therefore will not affect the health of the chicks, particularly in winter when, if the hatchling becomes wet through exposure to aqueous solution, the exposure may cause death of the hatchling. The use of the edible polysaccharide gel of the present invention which gels at a relatively low temperature is also suitable for adding nitrogen nutrients and other additives such as vitamins to the gel form or competitive exclusion products such as "BROILACT" sold by Orion Corp., Finland. This is especially useful with heat sensitive nutrients which, if exposed to temperatures over about 50°C, are denatured or inactivated. The use of the gel form vaccine of the present invention also realizes a saving in the hatchery in that as the gel form is 95 percent or more water, additional watering systems are not needed such as a built-in watering system. This not only reduces the costs of operation of the hatchery but also reduces the likelihood of the spilling of water and wetting of the chicks which can affect the health of the birds.

The use of the vaccine in accordance with the invention is illustrated in the following examples but the invention is not limited thereto. In all of the examples where it is stated that the animals were exposed to oocysts, it should be understood that the vaccine, being the oocysts in the gel form, was not fed to the bird manually, the birds were simply placed in the same area as the gel form vaccine during the time period and were allowed to consume the vaccine voluntarily. Eimeria tenella and Eimeria necatrix are used in the example because they are the two most pathogenic species among the six commonly found coccidia which causes coccidiosis in chickens. They are, however, the two least immunogenic species, that is, they produce the least protection against immunization. Hence, if an immunization method is effective against Eimeria tenella or Eimeria necatrix, it would be effective for the remaining species. Eimeria tenella is the preferred species for experimentation partly because it is more prevalent than Eimeria necatrix and is the main cause of death among chickens suffering from coccidiosis. In addition, because Eimeria tenella appears almost exclusively in the ceca of chickens instead of infecting all over the intestines, like other species, the damage or lesions can be accurately scored.

### Example 1

A vaccine according to the present invention was prepared by first adding 200 ml of hot tap water to 5 gm of kappa carrageenan Bengel MB 910® in a container and mixing until the MB 910 had dissolved. To this solution was added 200 ml of a solution containing 500 oocysts per ml of a mixture of Eimeria acervulina, E.maxima and E.tenella and the combined solution was mixed. The solution was then poured into a plastic watering dish and allowed to cool and gel at 4°C. This resulted in a gel form of the vaccine containing 1.25 percent MB 910 and 250 oocysts per ml.

### Example 2

33 broilers, each one day old, were divided into three groups of 11 birds each. Each group was immunized by exposure to Eimeria tenella by one of three methods. One group was immunized by use of a heavy dose spray cabinet, the second group was immunized by a heavy dose of oocysts suspended in a 1.5 percent carrageenan solution produced in accordance with Canadian Patent 1,204,057 and the third group was exposed to the gel form vaccine of the present invention prepared in accordance with Example 1 above. Each of the birds were weighed at the start and at the end to calculate the average weight gain and were also scored for lesions in the intestinal tract. The results of the experiment are set out in the following table:

**TABLE 1**

| | Ave. Weight Gain (g) | Presence of Lesions | | | Average Lesion Score | | |
|---|---|---|---|---|---|---|---|
| | | U | M | C | U | M | C |
| Spray Cabinet | 67 | 6/11 | 4/11 | 2/11 | 0.4 | 0.4 | 0.1 |
| | | | | | | | |
| Heavy Dose Liq. | 72 | 10/11 | 6/11 | 6/11 | 1.2 | 0.9 | 0.6 |
| | | | | | | | |
| Heavy Dose Gel | 77 | 11/11 | 11/11 | 6/11 | 1.3 | 1.2 | 0.6 |

The above results are utilized to illustrate the immunization of birds by the various forms of vaccine. Of most relevance to the immunizing capability of vaccine are the results of the lesions in the mid region of the gut, both the presence of lesions as well as the average lesions score. As can be seen, the gel form vaccine of the present invention provided complete immunization of the 11 birds as opposed to the only partial immunization of the group by the spray cabinet and the liquid administration methods.

### Example 3

480 Day old 8015 male broiler chickens were divided into four treatment groups and housed in a floor-pen house. Group A was a control group given a water placebo. Group B was given via gavage an aqueous suspension vaccine prepared in accordance with Canadian Patent 1,209,057 that included Eimeria acervulina, E. maxima and E. tenella, in a 1.5 percent carrageenan solution. Group C was given the gel form vaccine of the present invention prepared following the method of Example 1. Group D was given a water placebo and placed on low energy roaster starter feed medicated with 1.25 lbs of "MAXIBAN" feed additive-coccidiocide marketed by Elanco, a division of Eli Lilly Canada Inc. Groups A to C were placed on unmedicated feed. The birds were divided up into 4 pens of 30 birds (4 x 30 = 120 birds) for each treatment Group (A-D). At 29 days post immunization (PI), birds and feed from all Groups were weighed and feed was changed to low energy roaster grower. Group C continued to be maintained on medicated feed (1.00 lb "MAXIBAN"). Birds and feed were again measured at 59 days PI and feed was changed to roaster withdrawal feed. All groups received unmedicated feed during withdrawal until 70 days PI. The parameters of average weight gain, feed conversion, and mortality were measured for all cages at 29, 59 and 70 days PI. Lesion scores were done on 5 birds/pen (total of 20 birds/treatment group) on day 29.

**TABLE 2**

| | 4 Weeks | | 5 Weeks | | Lesion Score | | | 4 Wks |
|---|---|---|---|---|---|---|---|---|
| Treatment | Ave. Wt. | Ave.Wt. Gain | Ave. Wt. | Ave.Wt. Gain | Upper | Mid | Cecum | Feed Eff. |
| UI-UC Pens 1-4 | 914 | 869 | 1510 | 1459 | 0.08 | 1.00 | 0.20 | 1.66 |
| | | | | | | | | |
| Gavage I Pens 5-8 | 904 | 857 | 1500 | 1451 | 1.00 | 1.50 | 0.40 | 1.68 |
| | | | | | | | | |
| Gel I Pens 9-12 | 913 | 864 | 1490 | 1440 | 0.05 | 0.60 | 0.00 | 1.64 |
| | | | | | | | | |
| Medicated Pens 13-16 | 964 | 917 | 1570 | 1511 | 0.00 | 0.20 | 0.00 | 1.65 |
| UI=unimmunized | | | | | | | | |
| I=immunized | | | | | | | | |
| weight is in grams | | | | | | | | |

From the above results it can be seen that the weight gain and feed efficiency of all four groups is approximately equal.

The birds of Groups A to C were then challenged at three weeks with a challenge dose of 25,000 oocysts per bird and the parameters of weight gain, feed conversion and lesion scores were measured for all cages.

**TABLE 3**

| Treatment | Ave. Weight | Ave. Wt. Gain | Feed Conversion | Lesion Scores | | |
|---|---|---|---|---|---|---|
| | | | | Upper | Mid | Cecum |
| UI-UC | 865 | 304 | 1.62 | 0.0 | 0.0 | 0.0 |
| UI-C | 773 | 184 | 2.36 | 3.3 | 3.4 | 1.6 |
| I Gel-UC | 833 | 276 | 1.67 | 0.0 | 0.0 | 0.0 |
| I Gel-C | 826 | 203 | 2.06 | 2.5 | 3.0 | 0.8 |
| I Gavage-UC | 904 | 309 | 1.60 | 0.0 | 0.0 | 0.0 |
| I Gavage-C | 772 | 182 | 2.58 | 3.5 | 3.4 | 0.8 |
| Medicated-UC | 899 | 307 | 1.71 | 0.0 | 0.0 | 0.0 |
| UC=unchallenged | | | | | | |
| C=challenged | | | | | | |

From the results shown in Table 3, immunization with the gel form vaccine of the present invention provided better protection to the birds against the high dose oocyst challenge having lesion scores lower than those immunized by gavage. In addition, surprisingly, the feed conversion and weight gain of these birds was much better than that of the gavage immunized group.

### Example 4

The effectiveness of the gel form of the vaccine of the present invention was confirmed using flocks of commercial broilers. Flocks were either maintained on a medicated feed with Salinomycin (COXISTAC™) or Virginiamycin (STAFAC™) or were immunized utilizing a vaccine prepared according to Canadian Patent 1,204,057 or a gel form of the vaccine as prepared by a modified method according to Example 1. The gel form of the vaccine was prepared using a double strength oocysts suspension prepared in a 1.5% carrageenan solution as set out in Canadian Patent 1,204,057. The dissolved carrageenan MB 910 gel solution was prepared by dissolving the powder in 65°C water at a concentration of 2.5%. The two solutions were mixed together, poured into sausage sleeves and chilled with the gel forming in approximately 5 minutes. The sausage sleeves were sliced into 100 g slices with each slice designed for one tray or 100 birds. Consumption of the gel form was complete anywhere between 30 minutes and 2 hours. The effectiveness of the immunization by the gel form vaccine was investigated by examining ten fecal samples from each floor weekly by preparing a smear of the fecal sample and examining this under a microscope. The results of the fecal samples are as shown in Table 4.

**TABLE 4**

| Number of Positive Fecal Samples | | | |
|---|---|---|---|
| | Bottom Floor | Middle Floor | Top Floor |
| Week 1 | 2/10 | 3/9 | 4/9 |
| Week 2 | 7/9 | 5/9 | 8/10 |
| Week 3 | 10/10 | 11/11 | 10/10 |

As can be seen, by three weeks post-administration of the vaccine, there was complete immunization of the flock with all species of Eimeria present in all fecal samples.

Three weeks after immunization, 16 birds were removed from various pens, maintained separately and then challenged at 5 weeks post-immunization with heavy doses of E. tenella, E. acervuline and E. maxima. The results as shown in the following Table demonstrate the effectiveness of the vaccine. The average lesions in upper, middle guts and ceca were substantially higher in the control birds than in the vaccinated birds.

**TABLE 5**

| | | Average Lesion Score | | |
|---|---|---|---|---|
| | No. of Birds | Upper | Middle | Ceca |
| Control | 7* | 3.3 | 0.9 | 3.3,3.4 |
| Bottom Floor | 6 | 0 | 0 | 0.2,0.1 |
| Middle Floor | 6 | 0 | 0 | 0.3,0.2 |
| Top Floor | 6 | 0 | 0 | 0.1,0.2 |

| | | | | |
|---|---|---|---|---|
| * one bird died | | | | |

The birds were marketed at 41 days old and the birds immunized with the gel form vaccine had an average weight of 2.11 kg and a feed conversion of 1.91. The mortality of two flocks immunized with the gel form of the vaccine was 4.2% out of 46,818 cockerels placed and condemnation was 0.87%. The flock of 23,052 cockerels immunized by the hatchery method using the vaccine of Canadian Patent 1,204,057 had an average weight of 2.02 Kg and a fee conversion rate of 1.92. In contrast, four crops of birds totally 102,506 cockerels and pullets medicated with Salinomycin and Virginiamycin sold at 41 days had an average weight of 1.99 kg with a feed conversation of 1.88. These results are illustrated in the following table.

**TABLE 6**

| Date | No. of Birds Placed | Mortality (%) | Age (days) | Condm (%) | Avg. Wt KG (lbs) | FCR |
|---|---|---|---|---|---|---|
| MEDICATION | | | | | | |
| 7/94 | 28,050^{a} | 4.0 | 41 | 0.75 | 1.82(4.00) | 1.95 |
| 9/94 | 26,826^{b} | 3.9 | 41 | 0.47 | 2.08(4.58) | 1.86 |
| 11/94 | 24/578^{a} | 4.1 | 40 | 0.52 | 1.98(4.36) | 1.83 |
| 1/94 | 23,052^{a} | 3.5 | 41 | 0.82 | 2.08(4.58) | 1.88 |
| Average | | 3.9 | 40.8 | 0.64 | 1.99(4.39) | 1.88 |

| IMMUNIZATION | | | | | | |
|---|---|---|---|---|---|---|
| 3/95 | 23,052^{c} | 3.6 | 41 | 0.22 | 2.02(4.44) | 1.92 |
| 5/95 | 24,174^{d}* | 5.8 | 42 | 0.59 | 2.16(4.76) | 1.87 |
| 7/95 | 22,644^{d} | 2.5 | 40 | 1.14 | 2.06(4.54) | 1.94 |
| Average | | 4.2 | 41 | 0.87 | 2.11(4.65) | 1.91 |

| | | | | | | |
|---|---|---|---|---|---|---|
| a: Coxistac, 1/2 cockerels and 1/2 pullets | | | | | | |
| b: Coban | | | | | | |
| c: Hatchery method | | | | | | |
| d: Gel method | | | | | | |
| *: Staph infection within first week | | | | | | |

The gel form vaccine of the present invention provides for an easy to handle method of vaccinating poultry hatchlings in the hatchery and is, therefore, suitable for general hatchery workers without any special expertise required. The gel form is produced utilizing an edible polysaccharide gel preferably a temperature setting alginate or carrageenan gel, most preferably kappa carrageenan Bengel MB 910. The final concentration of the edible gel in the gel form is between 1 and 2.5 percent, preferably between 1 and 2 percent, more preferably between 1.25 and 1.75 percent and most preferably about 1.25 percent. The use of the edible polysaccharide gels of the present invention results in a gel which forms in about 3 to 5 minutes at 4°C which maintains the vaccine organisms in uniform suspension and allows for more uniform exposure of the poultry hatchlings to the vaccine organisms. The low content of the edible gum in the gel form means that approximately 95 percent or more of the gel form is water which can aid in the hydration of the bird and induce the feeding response.

## Claims

1. Use of a gel form of a live vaccine in the manufacture of a vaccine for administration of live organisms to poultry hatchlings, the gel form comprising approximately 1 to 2.5 % of a temperature setting edible polysaccharide gelling agent having suspended therein sufficient levels of live organism to provide for immunisation of a poultry hatchling flock.

2. Use as claimed in claim 1, **characterised in that** the live organisms are oocysts of *Eimeria*.

3. Use as claimed in claim 2, **characterised in that** the oocysts are selected from one or more of *Eimeria tenella, Eimeria necatrix, Eimeria acervulina, Eimeria maxima, Eimeria brunetti, Eimeria proecos, Eimeria mivati* and *Eimeria mitis*.

4. Use as claimed in claim claim3, **characterised in that** the oocysts are selected from one or more of *Eimeria tenella, Eimeria acervulina* and *Eimeria maxima.*

5. Use of claimed in any preceding claim, **characterised in that** the vaccine contains between about 100 and 500 oocysts per ml.

6. Use as claimed in any preceding claim, **characterised in that** the vaccine contains between about 200 and 300 oocysts per ml.

7. Use as claimed in any preceding claim, **characterised in that** the vaccine contain about 250 oocysts per ml.

8. Use as claimed in any preceding claim, **characterised in that** the vaccine contains about 1.25 % of an edible polysaccharide gelling agent.

9. Use as claimed in claim 8, **characterised in that** the edible polysaccharide gelling agent is kappa carrageenan.

10. Use as claimed in any preceding claim, **characterised in that** the gel form is a sliceable self-supporting gel form of a size to immunise a plurality of poultry hatchlings.

11. A process for the manufacture of a gel form of a live vaccine, the process comprising:-
• preparing a suspension of the viable organisms in cold water,
• dissolving an edible polysaccharide temperature dependent gelling agent in an aqueous solution at or about the melting point of the polysaccharide gelling agent,
• mixing the organism suspension and the gelling agent solution,
• pouring the mixture into a suitable container then allowing the mixture to cool and gel to produce a gelled live vaccine comprising from about 1 to about 2.5 % of an edible polysaccharide gel having suspended therein sufficient levels of the organism to provide immunisation to the flock
**characterised in** the use as a vaccine for administration of live organisms to poultry hatchlings.

12. A method as claimed in claim 11, **characterised in that** the live organisms are oocysts of *Eimeria.*

13. A method as claimed in claim 12, **characterised in that** the oocycts are selected from one or more of *Eimeria tenella, Eimeria necatrix, Eimeria acervulina, Eimeria maxima, Eimeria brunetti, Eimeria proecos, Eimeria mivati* and *Eimeria mitis*.

14. A method as claimed in claim 12, **characterised in that** the oocysts are selected from one or more of *Eimeria tenella, Eimeria acervulina* and *Eimeria maxima.*

15. A method as claimed in any preceding claim, **characterised in that** the vaccine contains between about 100 and 500 oocysts per ml.

16. A method as claimed in any preceding claim, **characterised in that** the vaccine contains between about 200 and 300 oocysts per ml.

17. A method as claimed in any preceding claim, **characterised in that** the vaccine contain about 250 oocysts per ml.

18. A method as claimed in any preceding claim, **characterised in that** the vaccine contains about 1.25 % of an edible polysaccharide gelling agent.

19. A method as claimed in claim 18, **characterised in that** the edible polysaccharide gelling agent is kappa carrageenan.

## Patentansprüche

1. Verwendung einer Lebendvakzine in Form eines Gels zur Herstellung einer Vakzine zur Verabreichung von lebenden Organismen an Geflügelküken, wobei die Gelform aus ungefähr 1 bis 2,5% eines temperaturabhängig gelierbaren, eßbaren Polysaccharid-Geliermittels besteht, enthaltend eine Suspension von ausreichenden Mengen an lebenden Organismen zur Immunisierung eines Geflügelkükenbestands.

2. Verwendung nach Anspruch 1, **dadurch gekennzeichnet, daß** die lebenden Organismen Oozyten von *Eimeria* sind.

3. Verwendung nach Anspruch 2, **dadurch gekennzeichnet, daß** die Oozyten ausgewählt sind aus einem oder mehreren von *Eimeria tenella, Eimeria necatrix, Eimeria acervulina, Eimeria maxima, Eimeria brunetti, Eimeria proecos, Eimeria mivati und Eimeria mitis*.

4. Verwendung nach Anspruch 3, **dadurch gekennzeichnet, daß** die Oozyten ausgewählt sind aus einem oder mehreren von *Eimeria tenella, Eimeria acervulina, Eimeria maxima*.

5. Verwendung nach einem der vorhergehenden Ansprüche, **dadurch gekennzeichnet, daß** die Vakzine zwischen rund 100 und 500 Oozyten pro ml enthält.

6. Verwendung nach einem der vorhergehenden Ansprüche, **dadurch gekennzeichnet, daß** die Vakzine zwischen rund 200 und 300 Oozyten pro ml enthält.

7. Verwendung nach einem der vorhergehenden Ansprüche, **dadurch gekennzeichnet, daß** die Vakzine rund 250 Oozyten pro ml enthält.

8. Verwendung nach einem der vorhergehenden Ansprüche, **dadurch gekennzeichnet, daß** die Vakzine ungefähr 1,25% eines temperaturabhängig gelierbaren, eßbaren Polysaccharid-Geliermittels enthält.

9. Verwendung nach Anspruch 8, **dadurch gekennzeichnet, daß** das temperaturabhängig gelierbare, eßbare Polysaccharid-Geliermittel kappa-Carrageenan ist.

10. Verwendung nach einem der vorhergehenden Ansprüche, **dadurch gekennzeichnet, daß** die Gelform ein schneidbares, selbsttragendes Gel ist, ausreichend zur Immunisierung einer Mehrzahl von Geflügelküken.

11. Verfahren zur Herstellung einer Lebendvakzine in Form eines Gels, umfassend die Verfahrensschritte:
• Vorbereiten einer Suspension lebensfähiger Organismen in kaltem Wasser,
• Lösen eines eßbaren, temperaturabhängig gelierbaren Polysaccharid-Geliermittels in einer wässrigen Lösung am oder beim Schmelzpunkt des Polysaccharid-Geliermittels,
• Vermischung der Organismen-Suspension und der Geliermittel-Lösung,
• Einbringung der Mischung in einen geeigneten Behälter, um der Mischung zu erlauben abzukühlen und um dem Gel zu erlauben eine gelierte Lebendvakzine zu produzieren, enthaltend ungefähr 1 bis ungefähr 2,5% eines eßbaren Polysaccharid-Gels, enthaltend darin suspendiert ausreichende Mengen der Organismen zur Immunisierung eines Geflügelkükenbestands
**gekennzeichnet durch** die Verwendung als Vakzine zur Verabreichung von lebenden Organismen an Geflügelküken.

12. Verfahren nach Anspruch 11, **dadurch gekennzeichnet, daß** die lebenden Organismen Oozyten von *Eimeria* sind.

13. Verfahren nach Anspruch 12, **dadurch gekennzeichnet, daß** die Oozyten ausgewählt sind aus einem oder mehreren von *Eimeria tenella, Eimeria necatrix, Eimeria acervulina, Eimeria maxima, Eimeria brunetti, Eimeria proecos, Eimeria mivati und Eimeria mitis*.

14. Verfahren nach Anspruch 12, **dadurch gekennzeichnet, daß** die Oozyten ausgewählt sind aus einem oder mehreren von *Eimeria tenella, Eimeria acervulina, Eimeria maxima.*

15. Verfahren nach einem der vorhergehenden Ansprüche, **dadurch gekennzeichnet, daß** die Vakzine zwischen rund 100 und 500 Oozyten pro ml enthält.

16. Verfahren nach einem der vorhergehenden Ansprüche, **dadurch gekennzeichnet, daß** die Vakzine zwischen rund 200 und 300 Oozyten pro ml enthält.

17. Verfahren nach einem der vorhergehenden Ansprüche, **dadurch gekennzeichnet, daß** die Vakzine rund 250 Oozyten pro ml enthält.

18. Verfahren nach einem der vorhergehenden Ansprüche, **dadurch gekennzeichnet, daß** die Vakzine ungefähr 1,25% eines temperaturabhängig gelierbaren, eßbaren Polysaccharid-Geliermittels enthält.

19. Verfahren nach Anspruch 18, **dadurch gekennzeichnet, daß** das temperaturabhängig gelierbare, eßbare Polysaccharid-Geliermittel kappa-Carrageenan ist.

## Revendications

1. Utilisation d'une forme de gel d'un vaccin vivant dans la fabrication d'un vaccin pour administration d'organismes vivants à des couvées de volailles, la forme de gel comprenant environ 1 à 2,5 % d'un agent gélifiant de polysaccharide comestible thermodurcissable dans lequel sont mis en suspension des taux suffisants d'organismes vivants pour effectuer l'immunisation d'une couvée de volailles.

2. Utilisation selon la revendication 1, **caractérisée en ce que** les organismes vivants sont des oocystes de *Eimeria*.

3. Utilisation selon la revendication 2, **caractérisée en ce que** les oocystes choisis sont un ou plusieurs parmi *Eimeria tenella, Eimeria necatrix, Eimeria acervulina, Eimeria maxima, Eimeria brunetti, Eimeria proecos, Eimeria mivati* et *Eimeria mitis*.

4. Utilisation selon la revendication 3, **caractérisée en ce que** les oocystes choisis sont un ou plusieurs parmi *Eimeria tenella, Eimeria acervulina* et *Eimeria maxima*.

5. Utilisation selon l'une quelconque des revendications précédentes, **caractérisée en ce que** le vaccin contient d'environ 100 à 500 oocystes par ml.

6. Utilisation selon l'une quelconque des revendications précédentes, **caractérisée en ce que** le vaccin contient d'environ 200 à 300 oocystes par ml.

7. Utilisation selon l'une quelconque des revendications précédentes, **caractérisée en ce que** le vaccin contient environ 250 oocystes par ml.

8. Utilisation selon l'une quelconque des revendications précédentes, **caractérisée en ce que** le vaccin contient environ 1,25 % d'un agent gélifiant de polysaccharide comestible.

9. Utilisation selon la revendication 8, **caractérisée en ce que** l'agent gélifiant de polysaccharide comestible est la carragénine kappa.

10. Utilisation selon l'une quelconque des revendications précédentes, **caractérisée en ce que** la forme de gel est une forme de gel solide pouvant être découpé en tranches d'une taille permettant d'immuniser une pluralité de couvées de volailles.

11. Procédé pour la fabrication d'une forme de gel d'un vaccin vivant, le procédé comprenant les étapes consistant à :
- préparer une suspension des organismes viables dans l'eau froide,
- dissoudre un agent gélifiant dépendant de la température de polysaccharide comestible dans une solution aqueuse au, ou environ au point de fusion de l'agent gélifiant de polysaccharide,
- mélanger la suspension d'organismes et la solution d'agent gélifiant,
- verser le mélange dans un récipient approprié puis laisser le mélange refroidir et gélifier pour produire un vaccin vivant gélifié comprenant d'environ 1 à environ 2,5 % d'un gel de polysaccharide comestible dans lequel sont mis en suspension des teneurs suffisantes de l'organisme pour produire l'immunisation des couvées
caractérisé dans l'utilisation en tant que vaccin pour administration d'organismes vivants à des couvées de volailles.

12. Procédé selon la revendication 11, **caractérisé en ce que** les organismes vivants sont des oocystes de *Eimeria*.

13. Procédé selon la revendication 12, **caractérisé en ce que** les oocystes choisis sont un ou plusieurs parmi *Eimeria tenella, Eimeria necatrix, Eimeria acervulina, Eimeria maxima, Eimeria brunetti, Eimeria proecos, Eimeria mivati* et *Eimeria mitis*.

14. Procédé selon la revendication 12, **caractérisé en ce que** les oocystes choisis sont un ou plusieurs parmi *Eimeria tenella, Eimeria acervulina* et *Eimeria maxima.*

15. Procédé selon l'une quelconque des revendications précédentes, **caractérisé en ce que** le vaccin contient d'environ 100 à 500 oocystes par ml.

16. Procédé selon l'une quelconque des revendications précédentes, **caractérisé en ce que** le vaccin contient d'environ 200 à 300 oocystes par ml.

17. Procédé selon l'une quelconque des revendications précédentes, **caractérisé en ce que** le vaccin contient environ 250 oocystes par ml.

18. Procédé selon l'une quelconque des revendications précédentes, **caractérisé en ce que** le vaccin contient environ 1,25 % d'un agent gélifiant de polysaccharide comestible.

19. Procédé selon la revendication 18, **caractérisé en ce que** l'agent gélifiant de polysaccharide comestible est la carragénine kappa.
